# EUROPEAN PATENT APPLICATION

(11) **EP 1 882 680 A1**
(43) Date of publication of application: **30.01.2008**
(21) Application number: 06746236.6
(22) Date of filing: 10.05.2006
(51) Int. Cl.: C07C 41/42, C07C 43/12, C07B 61/00

(54) **METHOD FOR PROCESSING FLUORINATED ALKYL ETHER**

(30) Priority: 17.05.2005 JP 2005144104
(71) Applicant: Asahi Glass Company, Limited, Chiyoda-ku, Tokyo 100-8405 (JP)
(72) Inventor: OKAMOTO, Hidekazu, Asahi Glass Company, Limited, Tokyo, 100-8405 (JP); SHIMIZU, Tamaki, Asahi Glass Company, Limited, Tokyo, 100-8405 (JP)
(74) Representative: Müller-Boré & Partner Patentanwälte
(86) International application number: PCT/JP2006/309426
(87) International publication number: WO 2006/123563

(57) **Abstract**

To provide a method for producing a high purity fluorinated alkyl ether having a very low content of an impurity having an unsaturated bond at the industrial scale.

A method for treating a fluorinated alkyl ether, which comprises bringing a reaction crude liquid containing a fluorinated alkyl ether obtained by reacting a fluorinated alkyl alcohol with a fluoroolefin in the presence of a basic catalyst, and an unsaturated impurity having an unsaturated bond formed as a by-product in the above reaction, into contact with a chlorine gas to convert the unsaturated impurity to a chlorine-added product, thereby to lower the content of the unsaturated impurity.

## Description

### TECHNICAL FIELD

The present invention relates to a technique to produce a fluorinated alkyl ether simply with high yield.

### BACKGROUND ART

A fluorinated alkyl ether is a compound which attracts attention in recent years as an alternative of a chlorofluorocarbon which has been used as CFC, and is characterized by having little influences over global environment such as ozone destruction and global warming, since it has a short lifetime in the atmosphere and has no chlorine atom.

Various methods have been reported as a method to prepare a fluorinated alkyl ether, and as a preparation method applicable at the industrial scale, an addition reaction of a fluorinated alkyl alcohol with a fluoroolefin in the presence of an alkali has been known (Patent Documents 1 and 2).

However, in such a reaction, since an alkali is used as a catalyst, dehydrohalogenation from the fluorinated alkyl ether as the aimed product or from the reaction intermediate simultaneously proceeds, and an impurity having an unsaturated bond will be formed as a by-product. Further, the impurity having an unsaturated bond has a boiling point close to that of the fluorinated alkyl ether, and accordingly it is difficult to obtain a high purity fluorinated alkyl ether by purification by distillation which is the industrially simplest purification method.

To overcome the above problem, a method of suppressing an impurity having an unsaturated bond by carrying out the reaction in a water solvent has been proposed (Patent Document 3). However, this method has such a problem that the reaction rate is remarkably inhibited, and further, it is difficult to suppress the content of an unsaturated impurity to be 150 ppm or lower.
Patent Document 1: U.S.P. 3,557,294
Patent Document 2: JP-A-9-263559
Patent Document 3: JP-A-2002-201152

### DISCLOSURE OF THE INVENTION

### OBJECT TO BE ACCOMPLISHED BY THE INVENTION

The object of the present invention is to provide a method for producing a high purity fluorinated alkyl ether having a very low content of an impurity having an unsaturated bond, at the industrial scale.

### MEANS TO ACCOMPLISH THE OBJECT

The present invention provides a method for treating a fluorinated alkyl ether, which comprises bringing a reaction crude liquid containing a fluorinated alkyl ether obtained by reacting a fluorinated alkyl alcohol with a fluoroolefin in the presence of a basic catalyst, and an unsaturated impurity having an unsaturated bond formed as a by-product in the above reaction, into contact with a chlorine gas to convert the unsaturated impurity to a chlorine-added product, and separating the chlorine-added product.

### EFFECTS OF THE INVENTION

According to the present invention, the above unsaturated impurity which is difficult to be separated by distillation, is converted to a chlorine-added product, which is easily removed by distillation. Thus, a high purity fluorinated alkyl ether can be produced at the industrial scale.

### BEST MODE FOR CARRYING OUT THE INVENTION

The reaction crude liquid in the present invention contains a fluorinated alkyl ether obtained by reacting a fluorinated alkyl alcohol with a fluoroolefin in the presence of a basic catalyst, and the above unsaturated impurity.

The unsaturated impurity is formed as a by-product when a fluorinated alkyl alcohol and the fluoroolefin are reacted to produce a fluorinated alkyl ether, and formed by dehydrohalogenation from the obtained fluorinated alkyl ether or from the reaction intermediate by the alkali used as a catalyst. For example, the unsaturated impurity may be a halogenated hydrocarbon having an unsaturated bond such as a double bond or a triple bond between carbon atoms. The unsaturated impurity does not include the fluoroolefin used as a raw material.

The fluorinated alkyl alcohol as the raw material in the present invention may be any compound so long as it is a compound having at least one hydrogen atom not in the hydroxyl group of a hydrocarbon alcohol substituted by a fluorine atom, and includes an alcohol having a fluorinated cycloalkyl group, but considering the industrial scale production and effects in the application field of the fluorinated alkyl ether as a product, preferred is a compound represented by.the formula 1:

R^{f}CH₂OH Formula 1

wherein R^{f} is -CₐH_{b}F_{d}Xₑ, X is a halogen atom other than a fluorine atom, each of "a" and "d" is an integer of at least 1, and each of "b" and "e" is an integer of at least 0, provided that b+d+e=2a+1.

In the formula 1, "a" is preferably an integer of from 1 to 10, particularly preferably an integer of from 2 to 4, from the viewpoint of availability. Further, "e" is preferably 0.

As the compound represented by the formula 1, specifically, a preferably used fluorinated alkyl alcohol may be CF₃CH₂OH, CF₃CF₂CH₂OH, CF₃(CF₂)₂CH₂OH, CF₃(CF₂)₃CH₂OH, CF₃(CF₂)₄CH₂OH, CF₃(CF₂)₅CH₂OH, CF₃(CF₂)₆CH₂OH, CHF₂CF₂CH₂OH, CHF₂(CF₂)₃CH₂OH, CHF₂(CF₂)₅CH₂OH, CF₃CHFCF₂CH₂OH or CHF₂CF(CF₃) CH₂OH.

A particularly preferred fluorinated alkyl alcohol may be 2,2,2-trifluoroethanol (CF₃CH₂OH, hereinafter referred to as TFEO) or 2,2,3,3-tetrafluoro-1-propanol (CHF₂CF₂CH₂OH, hereinafter referred to as TFPO).

Further, the fluoroolefin as another raw material in the present invention is preferably a compound represented by the formula 2:

CF₂=CYZ Formula 2

wherein each of Y and Z which are independent of each other, is a hydrogen atom, a fluorine atom or a trifluoromethyl group.

Specifically, the compound represented by the formula 2 may be CF₂=CF₂, CF₂=CHF, CF₂=CH₂ or CF₂=CFCF₃. The treatment method of the present invention is more effective particularly when CF₂=CFCF₃ (hexafluoropropene, hereinafter referred to as HFP) is used, since the amount of an unsaturated impurity as a by-product tends to be large.

The treatment method of the present invention is preferably applied to a reaction crude liquid containing a compound represented by the formula 3 (fluorinated alkyl ether) obtained by reacting the compound represented by the formula 1 (fluorinated alkyl alcohol) with HFP, and a compound represented by the formula 4 (the above unsaturated impurity) and/or a compound represented by the formula 5 (the above unsaturated impurity) which forms as a by-product in the above reaction:

R^{f}CH₂OCF₂CHFCF₃ Formula 3

R^{f}CH₂OCF₂CF=CF₂ Formula 4

R^{f}CH₂OCF=CFCF₃ Formula 5

wherein R^{f} is as defined for the formula 1.

Particularly, it is preferably applied to a reaction crude liquid containing 1,1,2,3,3,3-hexafluoro-1-(2,2,3,3-tetrafluoropropoxy)propane obtained by reacting TFPO with HFP, and 1,1,2,3,3-pentafluoro-3-(2,2,3,3-tetrafluoropropoxy)-1-propene (the above unsaturated impurity) and/or 1,2,3,3,3-pentafluoro-1-(2,2,3,3-tetrafluoropropoxy)-1-propene (the above unsaturated impurity) formed as a by-product in the reaction, or a reaction crude liquid containing 1,1,2,3,3,3-hexafluoro-1-(2,2,2-trifluoroethoxy)propane obtained by reacting TFEO with HFP, and 1,1,2,3,3-pentafluoro-3-(2,2,2-trifluoroethoxy)-1-propene (the above unsaturated impurity) and/or 1,2,3,3,3-pentafluoro-1-(2,2,2-trifluoroethoxy)-1-propene (the above unsaturated impurity) formed as a by-product in the reaction.

The basic catalyst to be used when the fluorinated alkyl alcohol and the fluoroolefin are reacted, is preferably an alkali metal alkoxide or an alkali metal hydroxide from the viewpoint of the degree of basicity and availability. The alkali metal alkoxide may be a commercially available product as it is, or may be one obtained by reacting an alkali metal, an alkali metal hydride or an alkali metal amide with an alcohol. The alcohol used in the above reaction is not particularly limited but is preferably a fluorinated alkyl alcohol to be used as the raw material in the present invention. Further, in the above reaction, the alkali metal may, for example, be Na, K or Cs, the alkali metal hydride may, for example, be NaH or KH, and the alkali metal amide may, for example, be NaNH₂ or KNH₂.

The alkali metal hydroxide is particularly preferably NaOH, KOH or the like from the viewpoint of handling efficiency and availability. Such an alkali metal hydroxide has such an advantage that it can be used in the form of an aqueous solution.

When the fluorinated alkyl alcohol and the fluoroolefin are reacted, a solvent may be used with a view to improving the reaction rate. The solvent is preferably an aprotic polar solvent, and it may be a linear ether such as diethyl ether or glyme, a cyclic ether such as dioxane or tetrahydrofuran, or a nitrile compound such as acetonitrile or propionitrile. Among them, a glyme such as tetraglyme is particularly preferred, with which the reaction rate will further improve, and which is easily separated from the product.

In a case where a solvent is used in the above reaction, the solvent is preferably separated and removed by distillation or the like before the reaction crude liquid is brought into contact with a chlorine gas, with a view to preventing corrosion of an apparatus and loss of chlorine by chlorination of the solvent.

In the present invention, the above unsaturated impurity is converted to a chlorine-added product by bringing the above reaction crude liquid into contact with a chlorine gas. For example, the compound represented by the formula 4 is converted to a compound represented by the formula 6, and the compound represented by the formula 5 is converted to a compound represented by the formula 7:

R^{f}CH₂OCF₂CClFCClF₂ Formula 6

R^{f}CH₂OCClFCClFCF₃ Formula 7

wherein R^{f} is as defined for the formula 1.

In the present invention, as a means of bringing the reaction crude liquid into contact with a chlorine gas, a vapor phase method in which a gasified reaction crude liquid and a chlorine gas are brought into contact with each other in a reactor filled with a catalyst such as active carbon, or a liquid phase method in which a chlorine gas is introduced to the reaction crude liquid under irradiation with light so that they are contacted with each other, may be mentioned.

The vapor phase method may be a method of carrying out a reaction phase with a catalyst in a fixed bed or a method of carrying out the reaction in a fluidized bed, and either method is applicable in the present invention. The type of the catalyst, the particle size, etc. are properly determined depending upon the reaction apparatus.

In a case where the treatment method of the present invention is carried out by a vapor phase method, the amount of the chlorine gas supplied is preferably such that the amount of the chlorine gas is from 1 to 1×10⁵ mol, particularly from 1 to 1×10⁴ mol, especially from 1 to 1×0³ mol per mol of the unsaturated impurity contained in the reaction crude liquid. If the amount of the chlorine gas supplied is too large, the aimed product may also be chlorinated in some cases.

When the reaction crude liquid and the chlorine gas are brought into contact, an inert gas component such as a nitrogen gas may coexist with a view to suppressing heat generation.

The temperature at which the reaction crude liquid and the chlorine gas are contacted is preferably such a temperature that the addition reaction of chlorine to the unsaturated impurity occurs but chlorination or decomposition of the fluorinated alkyl ether as the aimed product is suppressed. Specifically, it is preferably a temperature at which the fluorinated alkyl ether and chlorine present substantially as gases or higher, and at most 400°C, particularly preferably from 100 to 300°C.

The time over which the reaction crude liquid and the chlorine gas are contacted varies depending upon the type of the catalyst to be used and the flow rate of the chlorine gas to be brought into contact, but is preferably from 0.01 to 600 seconds, more preferably from 0.1 to 180 seconds.

The pressure during the above contact may be any pressure so long as the reaction crude liquid to be treated and the chlorine gas will not liquefied during the reaction, but is preferably slightly reduced pressure to 0.5 MPa (gauge pressure).

The catalyst to be used in the vapor phase method is preferably active carbon. Active carbon is not particularly limited so long as it adsorbs the fluoroolefin and chlorine, and is preferably one having a large surface area and excellent in acid resistance and halogen resistance. Specifically, it is preferably coconut shell active carbon, mineral active carbon, coal active carbon or petroleum active carbon. Since functional groups such as carbonyl groups present on the surface of the active carbon catalyst may impair catalytic activity, they are preferably removed by contact with a chlorine gas or the like.

In a case where the treatment reaction of the present invention is carried out in a liquid phase method, a chlorine gas is introduced to the reaction crude liquid under irradiation with light to bring them into contact with each other. A light source for light irradiation is preferably a light source capable of applying ultraviolet rays having a wavelength of about from 300 to about 400 nm. Specifically, it may, for example, be an arc lamp containing argon, mercury or xenon, or a filament lamp containing tungsten and a halogen.

The reaction temperature in the liquid phase method is preferably within a range of from about -50°C to about 200°C, particularly preferably from about -10°C to about 60°C. Further, the reaction pressure is usually preferably from atmospheric pressure to 1 MPa.

The chlorine gas may be continuously supplied under irradiation with light, or a predetermined amount may be introduced to the reactor all at once, and then light irradiation is started. The amount of the chlorine gas supplied is preferably such that the amount of the chlorine gas is from 1 to 10,000 mol, particularly from 1 to 1,000 mol, especially from 1 to 10 mol per mol of the unsaturated impurity contained in the reaction crude liquid. If the amount of the chlorine gas supplied is too large, the aimed product may also be chlorinated in some cases.

The content of the unsaturated impurity in the reaction crude liquid varies depending upon the concentration of the alkali used as a catalyst, the type of the raw materials and the solvent, the reaction conditions, etc., but is usually within a range of from 0.03 to 20 mass%. If the content of the unsaturated impurity exceeds the above range, it is preferred to preliminarily lower the total content of the above unsaturated impurity to be 20 mass% or lower by distillation or the like.

According to the treatment method of the present invention, the total content of the unsaturated impurity in the reaction crude liquid can be decreased to at most 150 ppm, more preferably at most 100 ppm.

In the treatment method of the present invention, it is preferred to bring the reaction crude liquid into contact with the chlorine gas to convert the unsaturated impurity to a chlorine-added product, and then to separate and remove the chlorine-added product by distillation. The distillation is carried out preferably by a distillation column with a large number of theoretical plate, and may be carried out either by continuous distillation or batch distillation. The pressure may be either atmospheric pressure or reduced pressure, but distillation is carried out preferably under reduced pressure with a view to suppressing formation of a decomposed product by heat.

### EXAMPLES

### EXAMPLE 1

825 g of TFEO and 570 g of 20 mass% KOH aqueous solution were charged into a pressure-resistant reactor having an internal capacity of 2.5 L, and oxygen in the reactor was removed by vacuum deaeration. The reactor was set in a warm water bath so that the internal temperature of the reactor would be 35°C, and HFP was continuously supplied to the reactor so that the internal pressure in the reactor would be constant at 0.2 MPa.
The reaction was continued until the amount of HFP supplied reached 1,240 g, and the reaction was completed when a predetermined amount of HFP was consumed. 2,631 g of organic phase A was recovered from the recovered reaction crude liquid. The recovered organic phase A was analyzed by gas chromatography (hereinafter referred to as GC analysis) and the results are shown in Table 1. Further, organic phase A was subjected to distillation with a distillation column with a number of theoretical plate of 1 to obtain 1,840 g of main fraction A. This main fraction A was subjected to GC analysis and the results are shown in Table 1.

**TABLE 1**

| | Organic phase A (area%) | Main fraction A (area%) |
|---|---|---|
| HFP | 1.4 | 0.1 |
| CF₃CH_{z}OCF₂CHFCF₃ (Aimed product) | 92 | 96 |
| CF₃CH₂OC₃F₅ (Unsaturated impurity) | 3 | 3.7 |
| Others | 3.6 | 0.2 |

Then, 1,000 g of the obtained main fraction A was charged into a 1 L three-necked flask made of glass equipped with a reflux condenser, and a chlorine gas was supplied at 100 mL/min for 35 minutes under UV irradiation by a 500 W high-pressure mercury lamp under cooling with ice, so that the main fraction and the chlorine gas are contacted.

The obtained liquid treated with chlorine was washed with water, and the composition of the recovered crude liquid B was subjected to GC analysis. The results are shown in Table 2. Further, crude liquid B was subjected to distillation with a distillation column with a number of theoretical plate of 10 to obtain 950 g of main fraction B. Main fraction B was subjected to GC analysis, and the results are shown in Table 2.

**TABLE 2**

| | Organic phase B (area%) | Main fraction B (area%) |
|---|---|---|
| CF₃CH₂OCF₂CHFCF₃ (Aimed product) | 95 | 99.9 |
| CF₃CH₂OC₃F₅ (Unsaturated impurity) | 0 | 0 |
| CF₃CH₂OC₃Cl₂F₅ (Chlorine-added product) | 3.7 | 0 |
| Others | 1.3 | 0.1 |

### EXAMPLE 2

A flow type reaction tube having an U-type reaction tube made of Inconel 600 having an inner diameter of 2.54 cm and a length of 600 cm, filled with 600 mL of active carbon catalyst (SHIRASAGI C2X, manufactured by Takeda Pharmaceutical Company Limited, ash content: 1.2 mass%), was immersed in an oil bath and maintained at 200°C. To the reaction tube, a nitrogen gas and a chlorine gas were supplied at 100 mL/min and at 880 mL/min, respectively, for 6 hours to remove unnecessary functional groups on the active carbon. Then, main fraction A obtained in Example 1 was gasified, and gasified main fraction A and a chlorine gas were supplied to the reaction tube at 300 mL/min and at 30 mL/min, respectively, so that they are contacted and reacted at 150°C. The obtained reaction gas was recovered in a trap cooled with dry ice.

1,000 g of main fraction A was treated, whereupon the reaction was terminated, and a crude liquid recovered in the above trap was washed with water to obtain crude liquid C having an acid component removed. The composition of the recovered crude liquid C was subjected to GC analysis and the results are shown in Table 3. Further, crude liquid C was subjected to distillation with a distillation column with a number of theoretical plate of 10 to obtain 950 g of main fraction C. Main fraction C was subjected to GC analysis, and the results are shown in Table 3.

**TABLE 3**

| GC composition | Crude liquid C (area%) | Main fraction C (area%) |
|---|---|---|
| CF₃CH₂OCF₂CHFCF₃ (Aimed product) | 96 | 99.9 |
| CF₃CH₂OC₃F₅ (Unsaturated impurity) | 0 | 0 |
| CF₃CH₂OC₃Cl₂F₅ (Chlorine-added product) | 3.7 | 0 |
| Others | 0.3 | 0.1 |

### EXAMPLE 3

1,007 g of TFPO, 540 g of a 20 mass% KOH aqueous solution and 50 g of tetraglyme were charged into a pressure-resistant reactor having an internal capacity of 2.5 L, and oxygen in the reactor was removed by vacuum deaeration. The reactor was set in a warm water bath so that the internal temperature of the reactor would be 35°C, and HFP was continuously supplied to the reactor so that the internal pressure in the reactor would be constant at 0.2 MPa. The reaction was continued until the amount of HFP supplied reached 1,146 g, and the reaction was completed when a predetermined amount of HFP was consumed. 2,734 g of organic phase D was recovered from the recovered reaction crude liquid. The recovered organic phase D was subjected to GC analysis and the results are shown in Table 4. Further, organic phase D was subjected to distillation with a distillation column with a number of theoretical plate of 1 to obtain 1,663 g of main fraction D. This main fraction D was subjected to GC analysis and the results are shown in Table 4.

**TABLE 4**

| | Organic phase D (area%) | Main fraction D (area%) |
|---|---|---|
| HFP | 3.7 | 0 |
| CHF₂CF₂CH₂OCF₂CHFCF₃ (Aimed product) | 82 | 96 |
| CHF₂CF₂CH₂OC₃F₅ (Unsaturated impurity) | 1.5 | 1.5 |
| Tetraglyme | 3.7 | 0 |
| Others | 9.1 | 2.5 |

Then, chlorination and post-treatment of 1,000 g of the obtained main fraction D were carried out in the same manner as in Example 2 to obtain crude liquid E. The composition of the recovered crude liquid E was subjected to GC analysis and the results are shown in Table 5. Further, crude liquid E was subjected to distillation with a distillation column with a number of theoretical plate of 10 to obtain 940 g of main fraction E. Main fraction E was subjected to GC analysis, and the results are shown in Table 5.

**TABLE 5**

| GC composition | Crude liquid E (area%) | Main fraction E (area%) |
|---|---|---|
| CHF₂CF₂CH₂OCF₂CHFCF₃ (Aimed product) | 96 | 99.9 |
| CHF₂CF₂CH₂OC₃F₅ (Unsaturated impurity) | 0 | 0 |
| CHF₂CF₂CH₂OC₃Cl₂F₅ (Chlorine-added product) | 1.5 | 0 |
| Others | 2.5 | 0.1 |

### INDUSTRIAL APPLICABILITY

A high purity fluorinated alkyl ether obtained by the present invention can be used for cleaning electronic components or as a solvent such as a lubricant or as a working fluid.

The entire disclosure of Japanese Patent Application No. 2005-144104 filed on May 17, 2005 including specification, claims, drawings and summary is incorporated herein by reference in its entirety.

## Claims

1. A method for treating a fluorinated alkyl ether, which comprises bringing a reaction crude liquid containing a fluorinated alkyl ether obtained by reacting a fluorinated alkyl alcohol with a fluoroolefin in the presence of a basic catalyst, and an unsaturated impurity having an unsaturated bond formed as a by-product in the above reaction, into contact with a chlorine gas to convert the unsaturated impurity to a chlorine-added product, and separating the chlorine-added product.

2. The method for treating a fluorinated alkyl ether according to Claim 1, wherein the fluorinated alkyl alcohol is a compound represented by the formula 1:
R^{f}CH₂OH Formula 1
wherein R^{f} is -CₐH_{b}F_{d}Xₑ, X is a halogen atom other than a fluorine atom, each of "a" and "d" is an integer of at least 1, and each of "b" and "e" is an integer of at least 0, provided that b+d+e=2a+1.

3. The method for treating a fluorinated alkyl ether according to Claim 1 or 2, wherein the fluoroolefin is a compound represented by the formula 2:
CF₂=CYZ Formula 2
wherein each of Y and Z which are independent of each other, is a hydrogen atom, a fluorine atom or a trifluoromethyl group.

4. The method for treating a fluorinated alkyl ether according to Claim 1 or 2, wherein the fluoroolefin is hexafluoropropene.

5. The method for treating a fluorinated alkyl ether according to any one of Claims 1 to 4, wherein the content of the unsaturated impurity in the reaction crude liquid before brought into contact with a chlorine gas, is from 0.03 to 20 mass%, and the content of the unsaturated impurity in the reaction crude liquid after brought into contact with a chlorine gas, is at most 150 ppm.

6. The method for treating a fluorinated alkyl ether according to any one of Claims 1 to 5, wherein after the reaction crude liquid is brought into contact with a chlorine gas to convert the unsaturated impurity in the reaction crude liquid to a chlorine-added product, the chlorine-added product is removed by distillation.
